# EUROPEAN PATENT APPLICATION

(11) **EP 3 275 479 A1**
(43) Date of publication of application: **31.01.2018**
(21) Application number: 16182094.9
(22) Date of filing: 29.07.2016
(51) Int. Cl.: A61M 5/178, G09F 3/02

(54) **SYRINGE LABELLING**

(71) Applicant: Tom H Williamson Ltd., Edinburgh, Lothian EH3 8JB (GB)
(72) Inventor: WILLIAMSON, Thomas Hardie, Esher, Surrey KT10 9JD (GB); GOODWIN, Edward John, Teddington, TW11 0SR (GB)
(74) Representative: O'Callaghan, Robert James

(57) **Abstract**

A flexible self-adhesive label (10), for a syringe (200) comprising a barrel (210) with a front end and a rear end and having a tip (220) at the front end of the barrel. The label comprises a main body (20), for labelling the barrel of the syringe, the main body having a front end and a rear end. The label further comprises a tongue (30) at the front end of the main body. The tongue includes an aperture (32) for engaging with the tip of the syringe, for locating the main body (20) of the label in a longitudinal direction with respect to the barrel. Also provided is a method of labelling and filling a syringe, using the label.

## Description

### FIELD OF THE INVENTION

This invention relates to a label for a syringe and a method of labelling and filling a syringe. It may be particularly relevant to the preparation of syringes for use in medical practice.

### BACKGROUND OF THE INVENTION

In medical practice, errors in administering medication or other substances can have grave consequences. Errors can include administering the wrong substance, or administering the wrong amount of a substance (or both). Misidentification of syringes is a particular problem. Usually, medicaments and other substances used in medical procedures are carefully labelled during storage (for example, in labelled vials or ampoules). However, when they are administered, they may be aspirated into generic and unlabelled syringes. It has been found in at least one study that the risk of actual administration of a wrongly selected drug is greater if the drug is in a syringe, rather than in an ampoule.

Generic syringes are also not well suited to the task of ensuring correct dosage. Typically, syringes are marked in standard units of volume, such as millilitres. When filling a syringe a doctor or nurse may need to calculate the correct dose for a patient. The correct dose may depend on the patient's weight and the correct volume to be administered may further depend on the concentration of the active drug in a given pharmaceutical product. Calculating the correct volume and filling the syringe with that correct volume is therefore prone to human error.

A particular problem arises in some cases, when it is necessary to mix or dilute a substance inside a syringe. This need arises, for example, in ocular surgery. It is common in vitreoretinal surgery to remove some or all of the vitreous gel from the eye and inject a gas in its place. The gases commonly used for this purpose include sulfur hexafluoride (SF₆), perfluoroethane (C₂F₆), and perfluoropropane (C₃F₈). Each of these is mixed with air, to a specified concentration that is different for each gas. The ranges of safe concentrations for C₃F₈ and SF₆ (at least) do not overlap.

The syringe is filled by firstly drawing the correct volume of the pure gas into the syringe, and then drawing air in to fill the remainder of the syringe and thereby dilute the gas concentration to the desired degree. As well as the usual problem of calculating the correct volume of gas and drawing that correct volume into the syringe, there is the additional problem that, after dilution with air, there is no way to check what volume of gas was originally drawn into the syringe. There is then neither a way to establish what gas is actually in the syringe, nor in what concentration that gas is present. A correctly filled syringe will look identical to an incorrectly filled syringe. Accordingly, there is no way to detect errors before the gas-mixture is administered. It would be particularly desirable to provide ways to reduce the likelihood of human error, in this context.

### SUMMARY OF THE INVENTION

The invention is defined by the claims.

According to one aspect, there is provided a flexible self-adhesive label, for a syringe comprising a barrel with a front end and a rear end and having a tip at the front end of the barrel, the label comprising:
a main body, for labelling the barrel of the syringe, the main body having a front end and a rear end; and
a tongue at the front end of the main body, the tongue including an aperture for engaging with the tip of the syringe, for locating the main body of the label in a longitudinal direction with respect to the barrel.

The main body of the label may be marked, shaped, or otherwise adapted so as to indicate to a user a desired volume of a specific fluid to be drawn into the syringe.

This can allow a syringe (which may be a standard, conventional, or generic syringe) to be marked in a consistent, reliable, and safe manner. It can enable the syringe to be customised for a specific purpose, in a way that reduces the likelihood of human error. The engagement of the tongue with the tip of the syringe tends to align the main body longitudinally on the barrel, thereby helping to ensure that the indication of the desired volume is provided at the correct location on the barrel.

In general, the aperture need not be completely enclosed by the tongue. For example, the aperture may comprise a slot or notch in the tongue. Alternatively or in addition, the tongue may comprise a yoke adapted to engage with the tip of the syringe. In some embodiments, the yoke may define a C-shaped, U-shaped, or V-shaped aperture in the tongue.

However, the aperture preferably comprises an opening in the tongue that is completely enclosed by the tongue.

That is, a contiguous piece of material of the tongue surrounds the opening so that the boundary of the opening defines a closed contour.

The opening may be circular.

The tongue is preferably shorter than the main body, in a longitudinal direction of the label.

The tongue is preferably narrower than the main body, in a transverse direction of the label.

The label optionally includes a tapering portion at the front end of the main body, where the main body meets the tongue.

The main body preferably includes opposing side edges that are straight and extend parallel to one another in a longitudinal direction of the label.

The label can have a straight rear edge at the rear end of the main body, said straight rear edge being parallel to a transverse direction of the label.

In use, the straight rear edge can preferably help to align the label with the rear end of the syringe-barrel. The barrel may have a flange at its rear end, to which the label rear edge can be aligned. Alternatively, the barrel may be marked with a scale and the rear edge of the label may align with the end of the scale. Alignment of the label with the rear end of the syringe-barrel increases safety still further, in combination with the engagement of the tongue with the tip of the syringe. For example, if the label is applied to a syringe of the wrong size, it is unlikely that both the tongue will be correctly engaged on the tip and the rear edge of the label will align with the rear end of the barrel. Thus, a further possible human error may be detected and avoided.

The label preferably further comprises a pair of ears extending transversely from the main body, at the rear end of the main body. The ears may be designed to meet each other once wrapped around the barrel of the syringe, thereby ensuring that they are applied to the correct diameter of syringe. Use on the wrong circumference of syringe will result in the ears overlapping or not meeting, providing an extra safety feature. Alternatively, for example on small diameter syringes, the opposing side edges of the stickers will be designed to meet once wrapped around the syringe.

Preferably, the ears and the rear end of the main body cooperate to define a straight rear edge lying parallel to a transverse direction of the label.

The ears may be shaped so as to align the label with a flange at the rear end of the barrel, or the end of a scale marked on the barrel. The ears may also help to prevent the main body of the label from peeling away from the barrel of the syringe, at least at the rear end.

The label may have a rear surface substantially entirely coated with adhesive, the label optionally being provided on a release-layer.

The release layer may comprise a silicone coated release paper.

The label preferably further comprises markings on the main body to indicate graphically a predefined volume of at least one predefined fluid to be drawn into the syringe.

For example, the markings may comprise a mark at a predetermined longitudinal position along the main body of the label, which indicates the longitudinal position in the syringe-barrel to which the plunger of the syringe should be pulled, in order to fill the syringe with the predefined volume of the fluid.

Further marks may be provided at different longitudinal positions, to indicate other different predefined volumes for other different predefined fluids.

The markings may be printed on the label.

Optionally, at least a portion of the main body of the label may be transparent. This can facilitate the user aligning the plunger of the syringe with the markings, when the label is in use on a syringe.

In some embodiments, the markings graphically indicate a longitudinal position and/or a longitudinal range of the barrel that corresponds to a safe dose of the predefined fluid.

When the fluid is drawn into the syringe (for example, from an ampoule or vial), the plunger is drawn to a position along the barrel that lies at the marked longitudinal position and/or lies within the graphically-indicated range. The syringe is thereby known (and can be seen) to contain a safe dose of the fluid.

Ranges and/or positions may also be indicated for different safe doses of other different fluids.

A marked longitudinal position may indicate a nominal correct dose. This may be derived theoretically from experimental models or may be, for example, an average dose used by surgeons in the procedure. The average dose may be a median dose.

The label preferably shows the name of the (or each) predefined fluid.

The fluid to be drawn into the syringe may be a gas.

The gas may be for use in ocular surgery, such as vitrectomy.

The aperture in the tongue may be adapted (for example, shaped) to engage with a Luer lock tip. Many standard syringes have a Luer lock tip, comprising a thread in the tip. In particular, when the fluid to be drawn into the syringe is a gas, such as for ocular surgery, it may be desirable to use a syringe with a Luer lock tip.

Also provided is a kit of parts, comprising: a syringe, comprising a barrel with a front end and a rear end, and having a tip at the front end of the barrel and a plunger at the rear end of the barrel; and a flexible self-adhesive label adapted to fit the syringe, as summarised above.

The label may be provided separately from the syringe or already affixed to it.

According to another aspect, there is provided a method of labelling a syringe and filling it with a predefined volume of a fluid, the syringe having a barrel, a tip, and a plunger, the method comprising:
providing a label as summarised above;
attaching the label to the syringe, by inserting the tip of the syringe through the aperture in the tongue of the label and attaching the main body of the label to the barrel of the syringe;
retracting the plunger until it aligns with the markings on the label, so as to draw the predefined volume of the fluid into the syringe.

Preferably, when attaching the label, the tip of the syringe is firstly inserted through the aperture in the tongue, and then the main body is attached to the side of the barrel by folding the tongue around the front end of the barrel.

According to an embodiment, there is provided a method of labelling a syringe and filling it with a mixture of gases, the syringe having a barrel, a tip, and a plunger, the method comprising:
providing a label as summarised above;
attaching the label to the syringe, by inserting the tip of the syringe through the aperture in the tongue of the label and attaching the main body of the label to the barrel of the syringe;
retracting the plunger until it aligns with the markings on the label, so as to draw a predefined volume of a first gas into the syringe; and
retracting the plunger further, to draw a second gas into the syringe and mix it with the first gas.

Also provided is a label for a syringe, optionally provided together with the syringe, substantially as described herein with reference to the accompanying drawings.

### BRIEF DESCRIPTION OF THE DRAWINGS

The invention will now be described by way of example with reference to the accompanying drawings, in which:
Fig. 1 illustrates a label according an embodiment of the invention;
Fig. 2 shows a syringe with which the label of Fig. 1 can be used; and
Fig. 3 shows the label of Fig. 1 attached to the syringe of Fig. 2.

It should be noted that these figures are diagrammatic and not drawn to scale. Relative dimensions and proportions of parts of these figures have been shown exaggerated or reduced in size, for the sake of clarity and convenience in the drawings.

### DETAILED DESCRIPTION

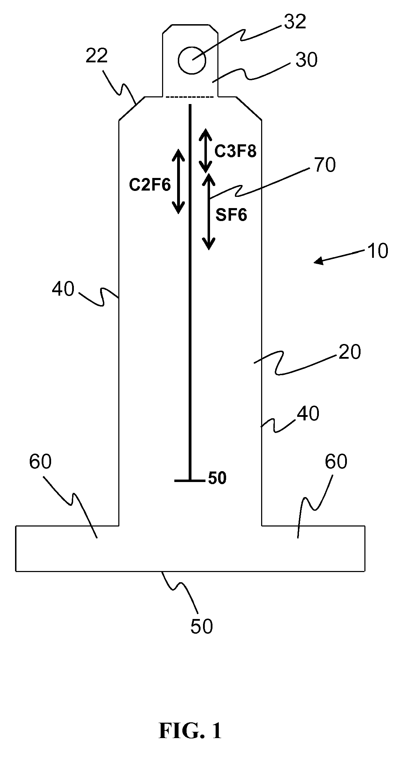

An exemplary embodiment of the invention will now be described with reference to Figs. 1-3.

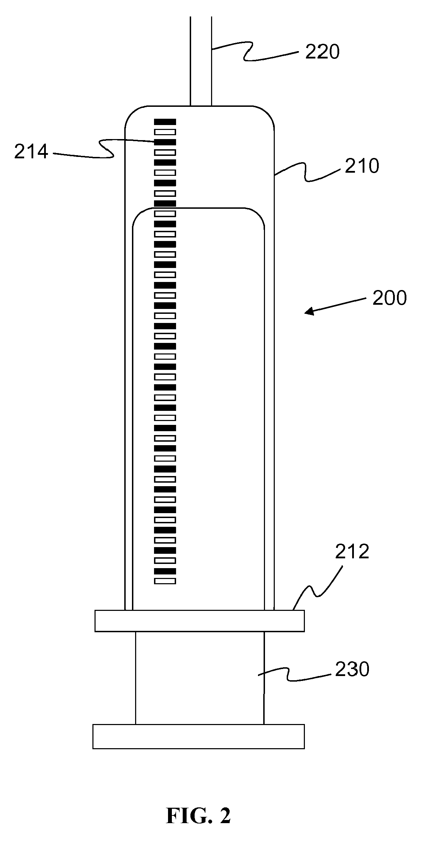

Fig. 1 shows a label 10 according to an embodiment of the invention. It comprises a main body 20, with a tongue 30 at the front end of the main body. The tongue 30 has a circular opening 32, which is adapted to engage with the tip of a syringe. One such syringe is shown in Fig. 2. The syringe 200 comprises a barrel 210, a tip 220, and a plunger 230. In general, the syringe 200 may be of a conventional type. It is envisaged that a suitable label 10 can be designed for substantially any type of syringe 200, by adapting the design of the label to the size and shape of that particular type of syringe 200.

In use, the circular opening 32 in the tongue 30 of the label 10 fits over the tip 220 of the syringe 200. For example, the tongue 30 may stick to the front end of the barrel 210, surrounding the tip 220. The label 10 is folded over the end of the barrel 210 so that the main body 20 of the label lies along the length of the outside of the barrel 210. The approximate location of the fold, in this example, is indicated by the dashed horizontal line in Fig. 1. In this way, the opening 32 in the tongue 30 serves to locate and align the main body 20 of the label 10 in the longitudinal direction with respect to the barrel 210 of the syringe 200. To the extent that the opening 32 engages closely with the tip 220, the longitudinal position of the main body 20 with respect to the barrel 210 can be controlled accurately.

In the present embodiment, the label 10 is a flexible, transparent label, with a self-adhesive backing extending over the whole rear surface of the label. This self-adhesive backing may be used to stick the label to the syringe 200.

As shown in Fig. 1, the tongue 30 is shorter and narrower than the main body 20. The label 10 also includes a tapering portion 22 at the front end of the main body 20, where the main body joins the tongue 30. This can enable the label to better match the shape of the syringe 200. The main body 20 has straight, parallel, opposing side edges 40. In use, these edges 40 may be aligned parallel to the longitudinal axis of the barrel 210. The rear edge 50 of the label is also straight and extends in a transverse direction with respect to the label - that is, the straight rear edge 50 is perpendicular to the straight side edges 40. Two ears 60 extend from the side edges 40 of the main body 20 in the transverse direction. The ears 60 are positioned at the rear end of the main body 20, such that the ears 60 terminate in the straight rear edge 50 of the label 10.

Markings 70 are provided on the main body 20 of the label 10. Because the tongue 30 serves to align the main body 20 longitudinally (as explained above), the markings 70 appear at a precise longitudinal position along the barrel 210, when the label 10 is in use on a syringe 200. The markings 70 can therefore indicate reliably a level to which the syringe should be filled. In the present example, the labelled syringe is intended for use in ocular surgery. The label 10 includes markings 70 relating to three gases: C₂F₆; C₃F₈; and SF₆. For each gas, the markings 70 indicate a safe range of volumes, by means of double-headed arrows aligned with the longitudinal direction of the barrel and label. As illustrated in Fig. 1, the safe range is different for each gas.

Alternatively, or in addition, markings may be provided indicating a specific dose to be administered, for one or more of the gases. This may be the correct theoretical dose. The "correct" dose may be derived either from experimental models or from prior surgical experience. For example, it may represent a median dose used by surgeons in previous (successful) procedures.

The label 10 can also include a mark on the main body 20 providing a numeric indication of a volume. In the example shown in Fig. 1, the label is marked with a line and the number "50", to indicate a level in the syringe corresponding to 50 ml. This can be useful in several ways. Firstly, a user can check to ensure that the 50 ml mark on the label 10 aligns with a 50 ml mark on a scale 214 provided on the syringe-barrel 210. This provides visual confirmation that the label 10 is aligned correctly on the syringe-barrel 210. It can also help to avoid a problem wherein the wrong type of label 10 is used on the syringe 200 (or, conversely, that the wrong type of syringe 200 is used with the label 10). Secondly, especially in the context of ocular surgery, the 50 ml mark on the label 10 can assist the user to dilute the gas in the syringe to a desired concentration by mixing with air.

The label 10 may be sold separately from the syringe 200 or both may be sold together. If sold together, they may be provided as separate parts or they may be provided with the label 10 already affixed to the syringe 200. In the event that the label 10 is provided separately from the syringe 200, the label 10 may be supplied on a silicone-coated release paper, to protect the adhesive on the back of the label.

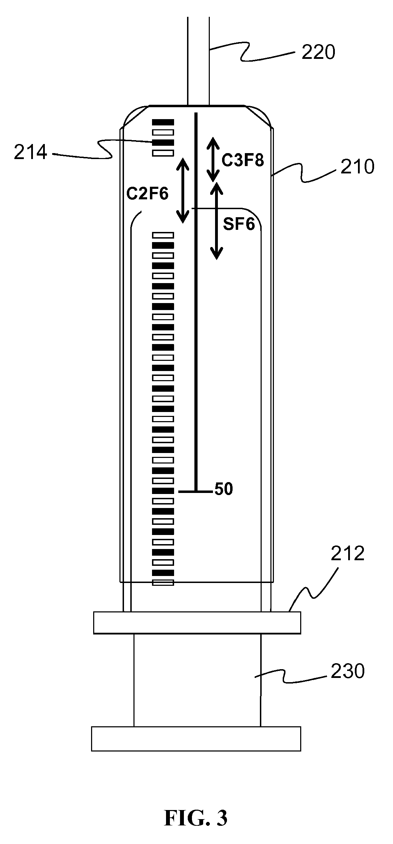

When used with the label 10, the syringe 200 may be labelled and filled as follows. The label 10 is peeled from the release paper (not shown). Next, the tip 220 of the syringe is inserted through the hole 32 in the tongue 30 of the label 10. The main body 20 of the label 10 is then folded about the end of the barrel 210 and stuck to the side surface of the barrel 210, to attach the main body 20 to the barrel 210. The syringe 200 can then be filled, using the markings 70 on the label 10 as a guide. Fig. 3 is representative of this stage in the process. In this example, it is assumed that the syringe is intended to be used to inject a mixture of C₂F₆ gas and air. Therefore, the tip of the syringe is inserted into a vial of C₂F₆ gas. To draw the correct volume of C₂F₆ gas in to the syringe, the plunger 230 is retracted until the front end of the plunger lies in the range indicated by the respective marking on the label 10. As shown in Fig. 3, a volume of C₂F₆ gas slightly less than the maximum safe volume has been drawn into the syringe 200. The tip 220 of the syringe 200 is then withdrawn from the vial of gas and air is drawn into the syringe by retracting the plunger 230 further. In particular, the plunger 230 is retracted until its front end aligns with the 50 ml mark on the label (and on the barrel 210). The user then knows that the correct volume of C₂F₆ gas is in the syringe and that this has been mixed with air to the correct concentration. This can avoid the need for the user to calculate volume or concentration values at the time that the syringe is being filled, with the associated risk of human error. For example, the correct concentration may be specified as a percentage but, in the example of Figs. 1 to 3, the syringe 200 can only hold about 50 ml of gas. In the past, the user would have needed to remember to divide the concentration value by a factor of two in order to calculate the correct volume of C₂F₆ gas for a 50 ml mixture. The user would then need to draw the calculated volume of gas into the syringe 200, using only the numerical scale 214 as a guide. There were several possibilities for error, in this approach, but these can be reduced by labelling the syringe in accordance with the present embodiment. By providing customised graphical indicators, the label 10 can avoid the need for the user to calculate concentrations or volumes, or count marks on a numerical scale 214, in order to draw a specified volume into the syringe. Thus, the process of filling the syringe is made simpler, more reliable, and less prone to human error.

In Fig. 3, the label 10 is shown in place on the syringe 200. The tongue 30 of the label is hidden, because it is folded out of sight on the front end of the barrel 210. Similarly, the ears 60 are not visible, because they have been wrapped around the side walls of the barrel 210. The rear edge 50 of the label 10 aligns with the end of the scale 214 on the barrel 210 of the syringe 200. This can provide an additional safety-check, because it allows the user to see that the correct-sized label 10 has been applied to the syringe 200. As an alternative, the rear edge 50 of the label 10 could align with the flange 212 at the rear end of the barrel 210, to achieve a similar purpose.

In the above description, the example was given of a 50 ml syringe. As will be apparent to those skilled in the art, this is not essential. Similar principles can be applied to label syringes of other sizes and shapes. A custom-label can be provided for each type of syringe, suitably adapted to match the size and shape of that syringe.

The markings 70 shown in Fig. 1 are just one example of the type of markings that may be provided on the label 10. The markings can conveniently be adapted to the size and shape of the syringe and also to the intended application. For example, the markings on the label may be personalised to an individual patient. A graphical mark may show the correct volume of a drug to be administered to a particular patient and the label may also have the patient's name or other identifying details printed on it. This can avoid the need to calculate the correct dose at the time that a drug (or other substance) is administered. In particular, it can avoid the need for calculations of dose that depend on the characteristics of the patient (such as age, weight, gender, etc.). This may be particularly beneficial in the case of drugs intended to be administered by the patient themselves.

In another example, the label 10 may include markings showing a numeric scale that is different from the scale 214 provided on the syringe-barrel 210. For example, for a given drug, a scale may be designed which shows the correct dose of that drug for a plurality of different patient-weights. A numeric scale in units of weight (mass) may be provided on the label. To provide the correct dose, a medical practitioner can weigh the patient to determine the correct weight value and then retract the plunger 230 of the syringe 200 until the end of the plunger aligns with the correct weight value on the scale provided on the label 10. Dosing in this way may be particularly advantageous in the field of paediatric medicine. In another example, dosages related to the age of the patient can be applied or other features such as the calorific content of the fluid be indicated.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

## Claims

1. A flexible self-adhesive label (10), for a syringe (200) comprising a barrel (210) with a front end and a rear end and having a tip (220) at the front end of the barrel, the label comprising:
a main body (20), for labelling the barrel of the syringe, the main body having a front end and a rear end; and
a tongue (30) at the front end of the main body, the tongue including an aperture (32) for engaging with the tip of the syringe, for locating the main body (20) of the label in a longitudinal direction with respect to the barrel.

2. The label of claim 1, wherein the aperture comprises an opening in the tongue that is completely enclosed by the tongue.

3. The label of claim 2, wherein the opening is circular.

4. The label of any preceding claim, wherein the tongue is shorter than the main body, in a longitudinal direction of the label.

5. The label of any preceding claim, wherein the tongue is narrower than the main body, in a transverse direction of the label.

6. The label of claim 5, wherein the label includes a tapering portion (22) at the front end of the main body, where the main body meets the tongue.

7. The label of any preceding claim, wherein the main body includes opposing side edges (40) that are straight and extend parallel to one another in a longitudinal direction of the label.

8. The label of any preceding claim, having a straight rear edge (50) at the rear end of the main body, said straight rear edge being parallel to a transverse direction of the label.

9. The label of any preceding claim, wherein the label further comprises a pair of ears (60) extending transversely from the main body, at the rear end of the main body.

10. The label of any preceding claim having a rear surface substantially entirely coated with adhesive, the label optionally being provided on a release-layer.

11. The label of any preceding claim, further comprising markings (70) on the main body to indicate graphically a predefined volume of at least one predefined fluid to be drawn into the syringe.

12. The label of claim 11, wherein the markings graphically indicate a longitudinal position and/or a longitudinal range of the barrel that corresponds to a safe dose of the predefined fluid.

13. The label of claim 12, wherein the fluid to be drawn into the syringe is a gas.

14. A kit of parts comprising:
a syringe (200), comprising a barrel (210) with a front end and a rear end, and having a tip (220) at the front end of the barrel and a plunger (230) at the rear end of the barrel; and
a flexible self-adhesive label (10) adapted to fit the syringe, according to any preceding claim.

15. A method of labelling a syringe (200) and filling it with a predefined volume of a fluid, the syringe having a barrel (210), a tip (220), and a plunger (230), the method comprising:
providing a label (10) according to claim 11, 12, or 13;
attaching the label (10) to the syringe (200), by inserting the tip (220) of the syringe (200) through the aperture (32) in the tongue (30) of the label and attaching the main body (20) of the label to the barrel (210) of the syringe; and
retracting the plunger (230) until it aligns with the markings (70) on the label, so as to draw the predefined volume of the fluid into the syringe.
